(19) Europäisches Patentamt
European Patent Office
Office européen des brevets

(11) Publication number: 0 488 196 A2

(12) # EUROPEAN PATENT APPLICATION

(21) Application number: 91120244.8

(22) Date of filing: 27.11.91

(51) Int. Cl.5: C12N 15/12, C12P 21/02, C07K 13/00, A61K 37/02, C12N 5/06

(30) Priority: 30.11.90 JP 330068/90
29.01.91 JP 8845/91

(43) Date of publication of application:
03.06.92 Bulletin 92/23

(84) Designated Contracting States:
AT BE CH DE DK ES FR GB GR IT LI LU NL SE

(71) Applicant: TAKEDA CHEMICAL INDUSTRIES, LTD.
1-1, Doshomachi 4-chome
Chuo-ku, OSAKA(JP)
Applicant: PRESIDENT OF NATIONAL CANCER CENTER
1-1, Tsukiji 5-chome, Chuo-ku
Tokyo 104(JP)

(72) Inventor: Terada, Masaaki
17-14, Sekimachiminami 2-chome, Nerima-ku
Tokyo 177(JP)
Inventor: Yoshida, Teruhiko
5-603, 7 Hikarigaoka 7-chome, Nerima-ku
Tokyo 179(JP)
Inventor: Sakamoto, Hiromi
2-24, Minamihanazono 2-chome
Chiba, Chiba 281(JP)
Inventor: Naito, Kenichiro
A503, 5 Hanjo 3-chome
Minoo, Osaka 562(JP)

(74) Representative: von Kreisler, Alek, Dipl.-Chem. et al
Patentanwälte Von Kreisler-Selting-Werner,
Deichmannhaus am Hauptbahnhof
W-5000 Köln 1(DE)

(54) **HST-2, a member of the heparin binding growth factor family.**

(57) Disclosed are (1) an HST2 protein; (2) a recombinant DNA containing a nucleotide sequence coding for the HST2 protein; (3) a vector containing the recombinant DNA; (4) a transformant transformed with the vector; and (5) a method for preparing the protein which comprises cultivating the transformant and accumulating the protein in a culture broth, whereby the HST2 protein can be produced in high purity and in large amounts which is applicable to wound healing, or repair of the cardiac muscle or the nervous tissue after ischemia.

EP 0 488 196 A2

## BACKGROUND OF THE INVENTION

The present invention relates to an HST2 protein, a recombinant DNA containing a nucleotide sequence coding for the HST2 protein, a vector containing the recombinant DNA, a transformant transformed with the vector, and a method for preparing the HST2 protein using the transformant.

Groups of cell growth factors belonging to the heparin-binding growth factor (HBGF) family show homology with one another on amino acid sequences and are commonly characterized by strong heparin-binding activity. The cell growth factors belonging to this family include acidic and basic fibroblast growth factors (aFGF and bFGF), HST1, INT2, FGF5 and keratinocyte growth factor (KGF). Of these cell growth factors, there is a report that FGF has activity to various cells and particularly has growth prompting activity and differentiation promoting activity to mesodermal cells [Gospodarowicz et al., J. Cell. Physiol., supplt. 5, 15 (1987)], and FGF has been investigated for application to drugs for promoting wound healing, or repair of the cardiac muscle or the nervous tissue after ischemia.

An HST1 gene is a transforming gene identified by a focus forming method, and a product thereof has cell growth promoting activity and angiogenesis inducing activity in vivo (Yoshida et al., submitted) similarly with FGF. An HST2 gene was identified as a gene having high homology with the HST1 gene by a DNA blot hybridization method [Sakamoto et al., Proc. Natl. Acad. Sci. USA, 83, 3997 (1986)]. Thus, the product of the HST2 gene has been determined by the present inventors as having activity similar to that of FGF or HST1 from the amino acid sequence homology. It is therefore an object to obtain the protein in large amounts and in high purity.

## SUMMARY OF THE INVENTION

The present inventors have made an important contribution to medical treatment and future studies by the present invention which provides recombinant technology for the preparation of the HST2 protein which has growth promoting activity for animal cells.

The present invention provides an HST2 protein; a recombinant DNA containing a nucleotide sequence coding for the HST2 protein; a vector containing the recombinant DNA; a transformant transformed with the vector; and a method for preparing the protein which comprises cultivating the transformant and accumulating the protein in a culture broth.

## BRIEF DESCRIPTION OF THE DRAWINGS

Fig. 1 is a simplified restriction enzyme map of cDNA cloned in cH2-5 obtained in Example 2, wherein abbreviations E, B and X represent EcoRI, BamHI and XhoI, respectively;

Fig. 2 shows a nucleotide sequence of a translation region of the cDNA cloned in pcH2-5 obtained in Example 2; and

Fig. 3 shows an amino acid sequence deduced from the nucleotide sequence shown in Fig. 2.

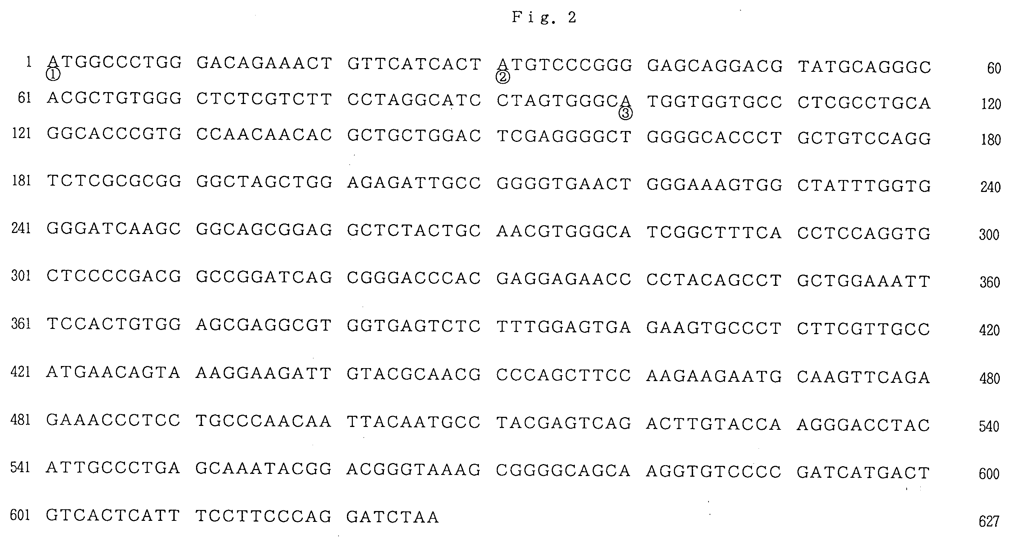

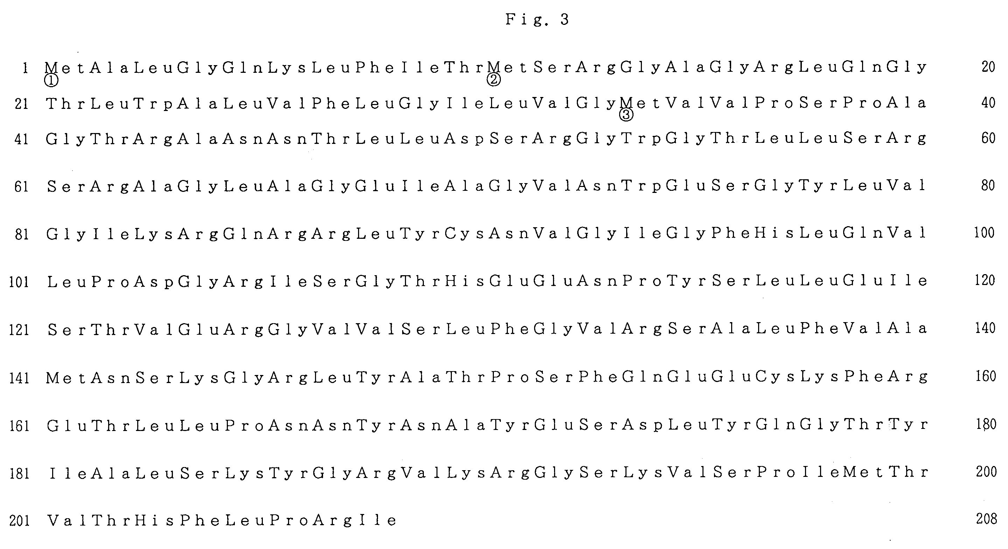

Referring to Figs. 2 and 3, (1), (2) and (3) show translation initiating points of three kinds of polypeptides.

## DESCRIPTION OF THE PREFERRED EMBODIMENTS

In Jpn. J. Cancer Res. Abstr. 500 (1989) the authors, which include co-inventors of the present invention, report that in restriction enzyme EcoRI fragments from human genomic DNA, as well as HST1, an 8kbp fragment was found which has similarity with ORF1 of HST1. The fragment was named "HST2". Cosmid clones containing HST1 or HST2 were obtained from a cosmid library made from normal human peripheral leucocytes as screened with ORF1 of HST1 gene as a probe. NIH3T3 cells were reported as being transfected with the clones, and focus forming assay and defined medium culture assay were applied to the cells. As a result, in the cells transfected with HST2 gene-containing clones, focus was not observed in the focus forming assay, but the cells which were morphologically-transformed appeared in the defined medium culture assay. Therefore, the authors conclude that HST2 might be a novel growth factor which belongs to the HST family. Neither amino acid sequence of HST2 nor any of its other chemical properties is reported in the foregoing abstract.

As the recombinant DNA of the present invention, any recombinant DNA may be used as long as it contains a nucleotide sequence coding for the HST2 protein. For example, DNA containing a nucleotide sequence coding for an amino acid sequence of Nos. 1 to 208 (SEQ ID NO: 1), Nos. 11 to 208 (SEQ ID NO: 2) or Nos. 34 to 208 (SEQ ID NO: 3) shown in Fig. 3 is preferably used. In particular, DNA containing, for example, a nucleotide sequence of Nos. 1 to 627 (SEQ ID NO: 4), Nos. 31 to 627 (SEQ ID NO: 5) or Nos. 100 to 627 (SEQ ID NO: 6) shown in Fig. 2 is preferred.

In Oncogene 5, 823 (1990), de Lapeyriere et al reported that the protein having growth promoting activity for the animal cells is possibly localized in the muscles or the testes. The leukemia cell strain HEL [Martin and Papayannoponlou, Science 216, 1223 (1982)] or CMK [Sato et al., British Journal of Hematology 72, 184 (1989)] which possibly produces the peptide have been reported. The isolation and purification of proteins having growth promoting activities is complicated and such proteins have heretofore been obtained only in small amounts.

The DNA of the present invention and the transformant thereof include the DNA sequence coding for the HST2 protein, and the HST2 protein used in the present method include the HST2 protein containing the amino acid sequence shown in Fig. 3 as a portion thereof.

In the present invention, an expression vector containing the nucleotide sequence coding for the HST2 protein can be prepared, for example, by the following process:

(i) RNA containing an HST2 sequence of the nucleotide sequence shown in Fig. 2 is isolated;
(ii) Complementary DNA (cDNA) is isolated from the RNA, followed by synthesis of double stranded DNA;
(iii) The complementary DNA is introduced into a phage or a plasmid;
(iv) The recombinant phage or plasmid thus obtained is introduced into an appropriate host cell to produce a transformant;
(v) After cultivation of the transformant thus obtained, the plasmid or the phage containing the desired DNA is isolated from the transformant by an appropriate method such as plaque hybridization using a DNA fragment obtained by restriction enzyme treatment of an HST2 gene as a probe;
(vi) The desired cloned DNA is cut out from the recombinant DNA; and
(v) The cloned DNA is ligated downstream from a promoter in a vector suitable for expression.

The RNA coding for the HST2 protein can be obtained, for example, from HST2-producing cells, chromosome DNA fragments themselves containing the HST2 genes, or from mouse cells transformed by cosmids or phage vectors into which the HST2 gene is introduced. Methods for preparing the RNA include the guanidine thiocyanate method [J. M. Chirgwin et al., Biochemistry 18, 5294 (1979)] and the like.

Using the RNA thus obtained as a template, cDNA is introduced into the plasmid or the phage by use of reverse transcriptase, for example, in accordance with the method of H. Okayama et al. [Mol. Cell. Biol. 2, 161 (1982); ibid. 3, 280 (1983)] or the method of U. Gubler and B. J. Hoffman [Gene 25, 263 (1983)].

The plasmids into which the cDNA is introduced include, for example, pBR322 [Gene 2,95 (1977)], pBR325 [Gene 4, 121 (1978)], pUC12 [Gene 19, 259 (1982)] and pUC13 [Gene 19, 259 (1982)], each derived from Escherichia coli, and pUB110 derived from Bacillus subtilis [Biochemical and Biophysical Research Communication 112, 678 (1983)]. However, any other plasmid can be used as long as it is replicable and viable in the host. The phage vectors into which the cDNA is introduced include, for example, λgt10 [R. Young and R. Davis, Proc. Natl. Acad. Sci., U.S.A. 80, 1194 (1983)]. However, any other phage vector can be used as long as it is replicable and viable in the host.

Methods for introducing the cDNA into the plasmid include, for example, the method described in T. Maniatis et al., Molecular Cloning, p.239, Cold Spring Laboratory, (1985). Methods for introducing the cDNA into the phage vector include, for example, the method of T. V. Hyunh et al. [DNA Cloning, A Practical Approach 1, 49 (1985)]. The plasmid and the phage vector thus obtained is introduced into the appropriate host cells such as E. coli.

Examples of E. coli described above include E. coli K12DH1 [Proc. Natl. Acad. Sci. U.S.A. 60, 160 (1968)], JM103 [Nucl. Acids Res. 9, 309 (1981)], JA221 [J. Mol. Biol. 120, 517, (1978)], HB101 [J. Mol. Biol. 41, 459 (1969)] and C600 [Genetics 39, 440 (1954)].

Examples of Bacillus subtilis described above include Bacillus subtilis MI114 [Gene 24, 255 (1983)] and 207-21 [J. Biochem. 95, 87 (1984)].

Methods for transforming the host with the plasmid include, for example, the calcium chloride method and the calcium chloride/rubidium chloride method described in T. Maniatis et al., Molecular Cloning, p.249, Cold Spring Harbor Laboratory, (1985).

The phage vector can be, for example, introduced to proliferated E. coli by the in vitro packaging method.

Desired clones are selected from the transformants thus obtained by known methods in the art such as the colony hybridization method [Gene 10, 63 (1980)], the plaque hybridization method [Science 196, 180 (1977)] and the DNA nucleotide sequence determination methods [Proc. Natl. Acad. Sci. U.S.A. 74, 560 (1977); Nucl. Acids Res. 9, 309 (1981)].

Thus, a cloned microorganism is obtained which contains the vector having the DNA containing the HST2 sequence.

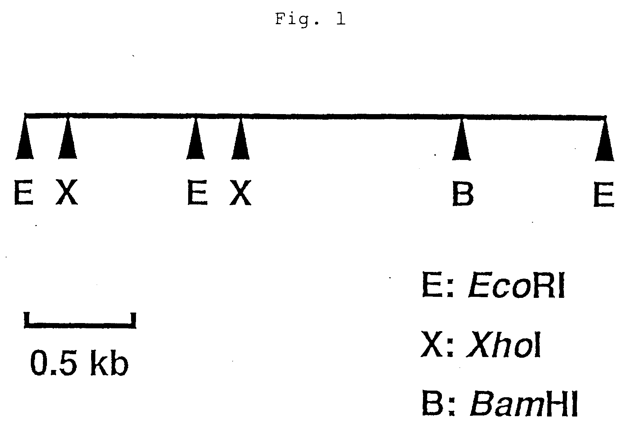

Plasmid pcH2-5 carried in E. coli HB101/cH2-5 obtained in Example 2 has the HST2 sequence. Fig. 1 shows restriction enzyme cleavage positions of the DNA. As shown in Fig. 1, the DNA has a whole length of about 2.7 Kbp, and is cleaved with restriction enzymes EcoRI, BamHI and XhoI to fragments.

Then, the plasmid or the phage vector is isolated from the microorganism.

Isolation methods include the alkali method [H. C. Birmboim et al., Nucl. Acids Res. 1, 1513 (1979)].

The plasmid or the phage vector having the DNA containing the nucleotide sequence coding for the above-mentioned cloned HST2 protein can be used as is or after digestion with a restriction enzyme if desired, depending on an intended use.

The cloned gene is ligated downstream from a promoter in a vehicle (vector) suitable for expression, whereby the expression vector can be obtained.

The vectors include the above plasmids derived from E. coli such as pBR322, pBR325, pUC12 and pUC13, plasmids derived from Bacillus subtilis such as pUB110, pTP5 and pC194, plasmids derived from yeast such as pSH19 and pSH15, bacteriophages such as λ phage, and animal viruses such as retroviruses and vaccinia viruses. The gene may have ATG as a translation initiating codon at the 5'-terminus thereof, and TAA, TGA or TAG as a translation terminating codon at the 3'-terminus thereof. A promoter is further ligated upstream therefrom to express the gene. As the promoter used in the present invention, any promoter is appropriate as long as it is suitable for expression in the host cell selected for the gene expression.

When the host cell used for transformation is E. coli, it is preferable that a trp promoter, a lac promoter, a rec promoter, a λPL promoter, a lpp promoter, etc. are used. When the host cell is Bacillus subtilis, it is preferable that an SP 01 promoter, an SP 02 promoter, a penP promoter, etc. are used. When the host cell is yeast, it is preferable that a PHO 5 promoter, a PGK promoter, a GAP promoter, an ADH promoter, etc. are used. In particular, it is preferable that the host cell is E. coli and the promoter is the trp promoter or the λPL promoter.

When the host cell is an animal cell, a SV40-derived promoter, a retrovirus promoter, etc. can be used. The SV40-derived promoter is particularly preferable.

By using a vector containing the DNA thus constructed, the transformant is prepared.

The host cells include procaryotes such as E. coli, Bacillus subtilis and actinomycetes, and eucaryotes such as yeast, molds and animal cells.

Specific examples of the above-mentioned E. coli and Bacillus subtilis include strains similar to those described above.

Specific examples of the above-mentioned yeast include Saccharomyces cerevisiae AH22, AH22R$^-$, NA87-11A and DKD-5D.

Specific examples of the above-mentioned animal cells include monkey cell COS-7, Vero, Chinese hamster cell (CHO), mouse cell and NIH 3T3 cell.

When bacterial, mold and yeast transformants are cultivated, a liquid medium is appropriate as a medium used for cultivation. Carbon sources, nitrogen sources, inorganic compounds and others necessary for growth of the transformants are contained therein. Examples of the carbon sources include glucose, dextrin, soluble starch and sucrose. Examples of the nitrogen sources include inorganic or organic materials such as ammonium salts, nitrates, corn steep liquor, peptone, casein, meat extracts, soybean meal and potato extract solution. The inorganic compounds include, for example, calcium chloride, sodium dihydrogenphosphate and magnesium chloride.

The pH of the medium is preferably about 5 to 8.

When the host cell is E. coli, it is preferred that the medium used is, for example, M9 medium containing glucose and Casamino Acids [Miller, J. Exp. Mol. Genet. p.431, Cold Spring Harbor Laboratory, New York (1972)]. The cultivation is usually carried out at about 14 to 43°C for about 3 to 24 hours with aeration or agitation if necessary.

When the host cell is Bacillus subtilis, the cultivation is usually carried out at about 30 to 40°C for about 6 to 24 hours with aeration or agitation if necessary.

When yeast transformants are cultivated, examples of the media include Burkholder minimum medium [K. L. Bostian, Proc. Natl. Acad. Sci. U.S.A. 77, 4505 (1980)]. The pH of the medium is preferably adjusted to about 5 to 8. The cultivation is usually carried out at about 20 to 35°C for about 24 to 72 hours with aeration or agitation if necessary.

When animal cell transformants are cultivated, examples of media which can be used include MEM medium containing about 5 to 20% fetal calf serum [Science, 122, 501 (1952)], DMEM medium [Virology, 8, 396 (1959)], RPMI 1640 medium [J. Am. Med. Assoc. 199, 519 (1967)] and 199 medium [Proc. Soc. Biol. Med. 73, 1 (1950)]. The pH is preferably about 6 to 8. The cultivation is usually carried out at about 30 to 40 ° C for about 15 to 60 hours, with aeration or agitation if necessary.

The HST2 protein of the present invention is produced and accumulated in or outside the cells. When the intracellular HST2 protein is to be extracted from the cultivated cells, the cells are collected by methods known in the art after cultivation. Then, the collected cells are suspended in a buffer solution containing a protein denaturant such as urea or guanidine hydrochloride, or a surface-active agent such as Triton X-100, followed by centrifugation to obtain a supernatant containing the HST2 protein, or the cells are disrupted by ultrasonic treatment, treatment by an enzyme such as lysozyme, or by freeze-thawing, followed by centrifugation to obtain a supernatant containing the HST2 protein. The separation and purification of the HST2 protein produced and accumulated in the supernatant or outside the cells can be carried out by an appropriate combination of known separating and purifying methods. These known separating and purifying methods include methods utilizing a difference in solubility such as salt precipitation and solvent precipitation, methods mainly utilizing a difference in molecular weight such as dialysis, ultrafiltration, gel filtration and SDS-polyacrylamide gel electrophoresis, methods utilizing a difference in electric charge such as ion-exchange chromatography, methods utilizing specific affinity such as affinity chromatography, methods utilizing a difference in hydrophobicity such as reverse phase high performance liquid chromatography, and methods utilizing a difference in isoelectric point such as isoelectro-focussing electrophoresis.

The HST2 protein of the present invention has the activity of promoting the growth of cells such as vascular endothelial cells and angiogenesis promoting activity, so that it can be used as drugs for treatment of burns, wounds and postoperative tissues, or repair of the cardiac muscle or the nervous tissue after ischemia. The HST2 protein of the present invention is low in toxicity. In addition, the HST2 protein of the present invention can be used as a reagent for promoting cell cultivation.

When the HST2 protein of the present invention is used as the drugs, it can be safely given parenterally or orally to warm-blooded animals (such as humans, mice, rats, hamsters, rabbits, dogs and cats), in a powder form as are or as pharmaceutical compositions (such as injections, tablets, capsules, solutions and ointments) with pharmaceutically acceptable carriers, excipients and diluents.

The injections are prepared by conventional methods using, for example, physiological saline or aqueous solutions containing glucose or other auxiliary agents. The pharmaceutical compositions such as tablets and capsules can also be prepared in accordance with conventional methods. When the injections, the solutions, the tablets and the capsules are prepared as the pharmaceutical compositions, they are prepared under aseptic conditions.

When the HST2 protein of the present invention is used as the drugs described above, it is given, for example, to the above-mentioned warm-blooded animals in an appropriate amount ranging from about 1 ng to 100 $\mu$g/kg daily, taking into account the routes of administration, symptoms, etc.

Further, when the HST2 protein of the present invention is used as the reagent for promoting cell cultivation, it is added to a medium preferably in an amount of about 0.01 to 10 $\mu$g per liter of medium, and more preferably in an amount of about 0.1 to 10 $\mu$g per liter of medium.

When nucleotides, amino acids and so on are indicated by abbreviations in the specification and drawings, the abbreviations adopted by the IUPAC-IUB Commission on Biochemical Nomenclature or commonly used in the art are employed. For example, the following abbreviations are used. When the amino acids are capable of existing as optical isomers, it is understood that the L-forms are represented unless otherwise specified.

| | |
|---|---|
| DNA : | Deoxyribonucleic acid |
| cDNA : | Complementary deoxyribonucleic acid |
| A : | Adenine |
| T : | Thymine |
| G : | Guanine |
| C : | Cytosine |
| RNA : | Ribonucleic acid |
| mRNA : | Messenger ribonucleic acid |
| dATP : | Deoxyadenosine triphosphate |
| dTTP : | Deoxythymidine triphosphate |
| dGTP : | Deoxyguanosine triphosphate |
| dCTP : | Deoxycytidine triphosphate |
| ATP : | Adenosine triphosphate |
| EDTA : | Ethylenediaminetetraacetic acid |
| SDS : | Sodium dodecyl sulfate |
| Gly or G : | Glycine |
| Ala or A : | Alanine |

Val or V : Valine
Leu or L : Leucine
Ile or I : Isoleucine
Ser or S : Serine
Thr or T : Threonine
Cys or C : Cysteine
Met or M : Methionine
Glu or E : Glutamic acid
Asp or D : Aspartic acid
Lys or K : Lysine
Arg or R : Arginine
His or H : Histidine
Phe or F : Phenylalanine
Tyr or Y : Tyrosine
Trp or W : Tryptophan
Pro or P : Proline
Asn or N : Asparagine
Gln or Q : Glutamine

The present invention will be described in more detail with the following Examples. It is understood of course that these Examples are not intended to limit the scope of the invention.

Transformed mouse cell strain 21SPLYH4D212 obtained in Example 1 was deposited with the Institute for Fermentation, Osaka, Japan (IFO) under the accession number IFO 50305 on December 10, 1990, and with the Fermentation Research Institute, the Agency of Industrial Science and Technology, the Ministry of International Trade and Industry, Japan (FRI) under the accession number FERM BP-3193 on December 11, 1990 under the Budapest Treaty.

Transformant E. coli HB101/cH2-5 obtained in Example 2 was deposited with the IFO under the accession number IFO 15119 on December 10, 1990, and with the FRI under the accession number FERM BP-3194 on December 11, 1990 under the Budapest Treaty.

Example 1

Preparation of Mouse Cell Transformed with Cosmid Vector LYH-4 Containing HST2 Gene and Preparation of RNA-Derived cDNA Library of The Transformed Cells

LYH-4 [Sakamoto et al., Biochem. Biophys. Res. Commun. 151, 965 (1988)] (In the literature reference, two clones having a HST2 gene, which were named LYH-4 and LYH-8, respectively, together with the clone LYH-3 are obtained.) was introduced into mouse NIH3T3 cells, and then transformed cells were selected using a serum-free medium in accordance with the method of Zhan et al. [Oncogene 1, 369 (1987)]. These transformed cells were further cloned by a limiting dilution method, and mouse cell strain 21SFLYH4D212 (IFO 50305, FERM BP-3193) transformed with LYH-4 was prepared.

RNA was extracted from the cells by the guanidine isothiocyanate method [J. M. Chirgwin et al., Biochemistry 18, 5294 (1979)]. Polyadenylated RNA was purified from the resulting RNA by oligo dT cellulose column chromatography [Aviv and Leder, Proc. Natl. Acad. Sci. U.S.A. 69, 1408 (1972)].

Using this poly(A+) RNA as a template, cDNA was purified by the method of U. Gubler and B. J. Hoffman [Gene 25, 263 (1983)], and then an EcoRI linker was added to the above cDNA in accordance with the method of T. V. Hyunh et al. [DNA Cloning I, A Practical Approach 1, 49 (1985)], followed by cloning to an EcoRI site of λgt10 to prepare a cDNA library.

Example 2

Isolation of Plasmid Containing HST2 cDNA and Determination of Nucleotide Sequence Thereof

E. coli NM514 was infected with the cDNA library of λgt10 obtained in Example 1, followed by inoculation onto a soft agar plate of L broth. After confirmation of appearance of plaques, positive clone λcH2-5 was selected by the plaque hybridization method [Science 196, 180 (1977)]. In this case, a LYH-4-derived PvuH/BamHI DNA fragment of 0.35 kbp was used as a probe. The whole length of the cDNA contained in HST2 is about 2.7 kbp, and a simplified restriction enzyme map thereof is shown in Fig. 1.

The cDNA was cut out of λcH2-5 with EcoRI, and subcloned into the EcoRI site of an M13mp18 phage to prepare M13mp18 cH2-5. E. coli JM109 was transformed with M13mp18 cH2-5 to prepare recombinant E. coli/M13mp18 cH2-5. M13mp18 cH2-5 was extracted from the resulting recombinant and purified by the alkali method [H. C. Birnboim and J. Doly, J. Nucl. Acids Res. 11, 1513 (1979)]. The nucleotide sequence of the cDNA portion of M13mp18 cH2-5 was determined by the method of F. Sanger et al. [Proc. Natl. Acad. Sci. U.S.A. 74, 5463 (1977)]. The nucleotide sequence of the translation region of the sequence thus determined is shown in Fig. 2, and the amino acid sequence deduced from this nucleotide sequence is shown in Fig.3.

The cDNA was cut out of λcH2-5 with EcoRI, and subcloned into the EcoRI site of plasmid (pGEM1) to prepare plasmid pcH2-5. E. coli HB101 was transformed with plasmid pcH2-5 to prepare transformant E. coli HB101/cH2-5 (IFO 15119, FERM BP-3194). pcH2-5 was extracted from the resulting recombinant and purified by the above-mentioned alkali method. The nucleotide sequence of the cDNA portion of pcH2-5 was determined by the above-mentioned method of F. Sanger et al. The nucleotide sequence of the translation region of the sequence thus determined is shown in Fig. 2, and the amino acid sequence deduced from this nucleotide sequence is shown in Fig.3.

## Example 3

Purification of HST2 Protein and Cell Growth Promoting Activity Thereof

Mouse cell 21SFLYH4D212 transformed with the HST gene obtained in Example 1, which was cultivated on a low-serum medium (1%) for 48 hours and was collected from 15 culture dishes having a diameter of 10 cm and suspended in the same amount of 10 mM Tris-HCl (pH 7.5), 1 M NaCl and 0.2% Triton X-100. The resulting suspension was subjected to ultrasonic treatment under ice cooling for 1 min., and a supernatant was obtained by centrifugation (15,000 rpm, 10 minutes) as a cell extract solution.

The cell extract solution was diluted with a 4-fold amount of 10 mM Tris-HCl (pH 7.5), and loaded on a heparin Sepharose (Pharmacia) column (0.5 ml). The column was sufficiently washed with 10 mM Tris-HCl (pH 7.5) and 0.2 M NaCl, and then column adsorption fractions were eluted increasing the concentration of NaCl stepwise from 0.5 to 1.5 M in 10 mM Tris-HCl (pH 7.5). The cell growth promoting activity of the eluted fractions was determined by the induction of DNA synthesis in mouse BALB/c 3T3 cells, based on [3H] thymidine uptake measured in accordance with the following method. The BALB/c 3T3 cells were suspended in DMEM medium containing 5% fetal calf serum to a concentration of $2X10^4$ cells/ml, and 1 ml of the suspension was added to a 24-well culture dish. After cultivation at 37°C for 24 hours, the serum concentration was reduced to 0.5% and cultivation was further continued for 72 hours. The eluted fractions from the heparin Sepharose column were added thereto, and 1 µCi of [3H] thymidine was added thereto after 16 hours. After 4 hours, the uptake of radioactive thymidine into acid-insoluble fractions was measured with a liquid scintillation counter. Strong cell growth promoting activity was observed in fractions eluted at 0.9 to 1.5 M NaCl, and the peak of the activity appeared in the vicinity of 1.3 M NaCl.

The DNA coding for the HST2 protein of the present invention can be used to produce the HST2 protein in high purity and in large amounts which is applicable to wound healing, or repair of the cardiac muscle or the nervous tissue after ischemia.

SEQUENCE LISTING

SEQ ID NO:1

LENGTH: 208 amino acids

TYPE: amino acid

TOPOLOGY: linear

MOLECULE TYPE: peptide

SEQUENCE DESCRIPTION:

```
Met Ala Leu Gly Gln Lys Leu Phe Ile Thr Met Ser Arg Gly Ala GLy
 1               5                  10                  15
Arg Leu Gln Gly Thr Leu Trp Ala Leu Val Phe Leu Gly Ile Leu Val
            20                  25                  30
Gly Met Val Val Pro Ser Pro Ala Gly Thr Arg Ala Asn Asn Thr Leu
        35                  40                  45
Leu Asp Ser Arg Gly Trp Gly Thr Leu Leu Ser Arg Ser Arg Ala Gly
    50                  55                  60
Leu Ala Gly Glu Ile Ala Gly Val Asn Trp Glu Ser Gly Tyr Leu Val
65                  70                  75                  80
Gly Ile Lys Arg Gln Arg Arg Leu Tyr Cys Asn Val Gly Ile Gly Phe
                85                  90                  95
His Leu Gln Val Leu Pro Asp Gly Arg Ile Ser Gly Thr His Glu Glu
            100                 105                 110
Asn Pro Tyr Ser Leu Leu Glu Ile Ser Thr Val Glu Arg Gly Val Val
        115                 120                 125
Ser Leu Phe Gly Val Arg Ser Ala Leu Phe Val Ala Met Asn Ser Lys
    130                 135                 140
Gly Arg Leu Tyr Ala Thr Pro Ser Phe Gln Glu Glu Cys Lys Phe Arg
145                 150                 155                 160
```

Glu Thr Leu Leu Pro Asn Asn Tyr Asn Ala Tyr Glu Ser Asp Leu Tyr
165 170 175
Gln Gly Thr Tyr Ile Ala Leu Ser Lys Tyr Gly Arg Val Lys Arg Gly
180 185 190
Ser Lys Val Ser Pro Ile Met Thr Val Thr His Phe Leu Pro Arg Ile
195 200 205

SEQ ID NO:2
LENGTH: 198 amino acids
TYPE: amino acid
TOPOLOGY: linear
MOLECULE TYPE: peptide
SEQUENCE DESCRIPTION:
Met Ser Arg Gly Ala Gly Arg Leu Gln Gly Thr Leu Trp Ala Leu Val
1 5 10 15
Phe Leu Gly Ile Leu Val Gly Met Val Val Pro Ser Pro Ala Gly Thr
20 25 30
Arg Ala Asn Asn Thr Leu Leu Asp Ser Arg Gly Trp Gly Thr Leu Leu
35 40 45
Ser Arg Ser Arg Ala Gly Leu Ala Gly Glu Ile Ala Gly Val Asn Trp
50 55 60
Glu Ser Gly Tyr Leu Val Gly Ile Lys Arg Gln Arg Arg Leu Tyr Cys
65 70 75 80
Asn Val Gly Ile Gly Phe His Leu Gln Val Leu Pro Asp Gly Arg Ile
85 90 95
Ser Gly Thr His Glu Glu Asn Pro Tyr Ser Leu Leu Glu Ile Ser Thr
100 105 110

Val Glu Arg Gly Val Val Ser Leu Phe Gly Val Arg Ser Ala Leu Phe
115 120 125

Val Ala Met Asn Ser Lys Gly Arg Leu Tyr Ala Thr Pro Ser Phe Gln
130 135 140

Glu Glu Cys Lys Phe Arg Glu Thr Leu Leu Pro Asn Asn Tyr Asn Ala
145 150 155 160

Tyr Glu Ser Asp Leu Tyr Gln Gly Thr Tyr Ile Ala Leu Ser Lys Tyr
165 170 175

Gly Arg Val Lys Arg Gly Ser Lys Val Ser Pro Ile Met Thr Val Thr
180 185 190

His Phe Leu Pro Arg Ile
195

SEQ ID NO:3

LENGTH: 175 amino acids

TYPE: amino acid

TOPOLOGY: linear

MOLECULE TYPE: peptide

SEQUENCE DESCRIPTION:

Met Val Val Pro Ser Pro Ala Gly Thr Arg Ala Asn Asn Thr Leu Leu
1 5 10 15

Asp Ser Arg Gly Trp Gly Thr Leu Leu Ser Arg Ser Arg Ala Gly Leu
20 25 30

Ala Gly Glu Ile Ala Gly Val Asn Trp Glu Ser Gly Tyr Leu Val Gly
35 40 45

Ile Lys Arg Gln Arg Arg Leu Tyr Cys Asn Val Gly Ile Gly Phe His
50 55 60
Leu Gln Val Leu Pro Asp Gly Arg Ile Ser Gly Thr His Glu Glu Asn
65 70 75 80
Pro Tyr Ser Leu Leu Glu Ile Ser Thr Val Glu Arg Gly Val Val Ser
85 90 95
Leu Phe Gly Val Arg Ser Ala Leu Phe Val Ala Met Asn Ser Lys Gly
100 105 110
Arg Leu Tyr Ala Thr Pro Ser Phe Gln Glu Glu Cys Lys Phe Arg Glu
115 120 125
Thr Leu Leu Pro Asn Asn Tyr Asn Ala Tyr Glu Ser Asp Leu Tyr Gln
130 135 140
Gly Thr Tyr Ile Ala Leu Ser Lys Tyr Gly Arg Val Lys Arg Gly Ser
145 150 155 160
Lys Val Ser Pro Ile Met Thr Val Thr His Phe Leu Pro Arg Ile
165 170 175

SEQ ID NO:4
LENGTH: 627 base pairs
TYPE: nucleic acid
STRANDEDNESS: double
TOPOLOGY: linear
MOLECULE TYPE: cDNA
SEQUENCE DESCRIPTION:

ATGGCCCTGG GACAGAAACT GTTCATCACT ATGTCCCGGG GAGCAGGACG TCTGCAGGGC 60
ACGCTGTGGG CTCTCGTCTT CCTAGGCATC CTAGTGGGCA TGGTGGTGCC CTCGCCTGCA 120
GGCACCCGTG CCAACAACAC GCTGCTGGAC TCGAGGGGCT GGGGCACCCT GCTGTCCAGG 180
TCTCGCGCGG GGCTAGCTGG AGAGATTGCC GGGGTGAACT GGGAAAGTGG CTATTTGGTG 240

GGGATCAAGC GGCAGCGGAG GCTCTACTGC AACGTGGGCA TCGGCTTTCA CCTCCAGGTG 300
CTCCCCGACG GCCGGATCAG CGGGACCCAC GAGGAGAACC CCTACAGCCT GCTGGAAATT 360
TCCACTGTGG AGCGAGGCGT GGTGAGTCTC TTTGGAGTGA GAAGTGCCCT CTTCGTTGCC 420
ATGAACAGTA AAGGAAGATT GTACGCAACG CCCAGCTTCC AAGAAGAATG CAAGTTCAGA 480
GAAACCCTCC TGCCCAACAA TTACAATGCC TACGAGTCAG ACTTGTACCA AGGGACCTAC 540
ATTGCCCTGA GCAAATACGG ACGGGTAAAG CGGGGCAGCA AGGTGTCCCC GATCATGACT 600
GTCACTCATT TCCTTCCCAG GATCTAA 627

SEQ ID NO:5

LENGTH: 597 base pairs

TYPE: nucleic acid

STRANDEDNESS: double

TOPOLOGY: linear

MOLECULE TYPE: cDNA

SEQUENCE DESCRIPTION:

ATGTCCCGGG GAGCAGGACG TCTGCAGGGC ACGCTGTGGG CTCTCGTCTT CCTAGGCATC 60
CTAGTGGGCA TGGTGGTGCC CTCGCCTGCA GGCACCCGTG CCAACAACAC GCTGCTGGAC 120
TCGAGGGGCT GGGGCACCCT GCTGTCCAGG TCTCGCGCGG GGCTAGCTGG AGAGATTGCC 180
GGGGTGAACT GGGAAAGTGG CTATTTGGTG GGGATCAAGC GGCAGCGGAG GCTCTACTGC 240
AACGTGGGCA TCGGCTTTCA CCTCCAGGTG CTCCCCGACG GCCGGATCAG CGGGACCCAC 300
GAGGAGAACC CCTACAGCCT GCTGGAAATT TCCACTGTGG AGCGAGGCGT GGTGAGTCTC 360
TTTGGAGTGA GAAGTGCCCT CTTCGTTGCC ATGAACAGTA AAGGAAGATT GTACGCAACG 420
CCCAGCTTCC AAGAAGAATG CAAGTTCAGA GAAACCCTCC TGCCCAACAA TTACAATGCC 480
TACGAGTCAG ACTTGTACCA AGGGACCTAC ATTGCCCTGA GCAAATACGG ACGGGTAAAG 540
CGGGGCAGCA AGGTGTCCCC GATCATGACT GTCACTCATT TCCTTCCCAG GATCTAA 597

SEQ ID NO:6

LENGTH: 528 base pairs

TYPE: nucleic acid

STRANDEDNESS: double

TOPOLOGY: linear

MOLECULE TYPE: cDNA

SEQUENCE DESCRIPTION:

```
ATGGTGGTGC CCTCGCCTGC AGGCACCCGT GCCAACAACA CGCTGCTGGA CTCGAGGGGC    60
TGGGGCACCC TGCTGTCCAG GTCTCGCGCG GGGCTAGCTG GAGAGATTGC CGGGGTGAAC   120
TGGGAAAGTG GCTATTTGGT GGGGATCAAG CGGCAGCGGA GGCTCTACTG CAACGTGGGC   180
ATCGGCTTTC ACCTCCAGGT GCTCCCCGAC GGCCGGATCA GCGGGACCCA CGAGGAGAAC   240
CCCTACAGCC TGCTGGAAAT TTCCACTGTG GAGCGAGGCG TGGTGAGTCT CTTTGGAGTG   300
AGAAGTGCCC TCTTCGTTGC CATGAACAGT AAAGGAAGAT TGTACGCAAC GCCCAGCTTC   360
CAAGAAGAAT GCAAGTTCAG AGAAACCCTC CTGCCCAACA ATTACAATGC CTACGAGTCA   420
GACTTGTACC AAGGGACCTA CATTGCCCTG AGCAAATACG GACGGGTAAA GCGGGGCAGC   480
AAGGTGTCCC CGATCATGAC TGTCACTCAT TTCCTTCCCA GGATCTAA                528
```

## Claims

1. A substantially purified HST2 protein having an amino acid sequence of SEQ ID NO:1:

Met Ala Leu Gly Gln Lys Leu Phe Ile Thr Met Ser Arg Gly Ala GLy Arg Leu Gln Gly Thr Leu Trp Ala Leu Val Phe Leu Gly Ile Leu Val Gly Met Val Val Pro Ser Pro Ala Gly Thr Arg Ala Asn Asn Thr Leu Leu Asp Ser Arg Gly Trp Gly Thr Leu Leu Ser Arg Ser Arg Ala Gly Leu Ala Gly Glu Ile Ala Gly Val Asn Trp Glu Ser Gly Tyr Leu Val Gly Ile Lys Arg Gln Arg Arg Leu Tyr Cys Asn Val Gly Ile Gly Phe His Leu Gln Val Leu Pro Asp Gly Arg Ile Ser Gly Thr His Glu Glu Asn Pro Tyr Ser Leu Leu Glu Ile Ser Thr Val Glu Arg Gly Val Val Ser Leu Phe Gly Val Arg Ser Ala Leu Phe Val Ala Met Asn Ser Lys Gly Arg Leu Tyr Ala Thr Pro Ser Phe Gln Glu Glu Cys Lys Phe Arg Glu Thr Leu Leu Pro Asn Asn Tyr Asn Ala Tyr Glu Ser Asp Leu Tyr Gln Gly Thr Tyr Ile Ala Leu Ser Lys Tyr Gly Arg Val Lys Arg Gly Ser Lys Val Ser Pro Ile Met Thr Val Thr His Phe Leu Pro Arg Ile,

SEQ ID NO:2:

Met Ser Arg Gly Ala Gly Arg Leu Gln Gly Thr Leu Trp Ala Leu Val Phe Leu Gly Ile Leu Val Gly Met Val Val Pro Ser Pro Ala Gly Thr Arg Ala Asn Asn Thr Leu Leu Asp Ser Arg Gly Trp Gly Thr Leu Leu Ser Arg Ser Arg Ala Gly Leu Ala Gly Glu Ile Ala Gly Val Asn Trp Glu Ser Gly Tyr Leu Val Gly Ile Lys Arg Gln Arg Arg Leu Tyr Cys Asn Val Gly Ile Gly Phe His Leu Gln Val Leu Pro Asp Gly Arg Ile Ser Gly Thr His Glu Glu Asn Pro Tyr Ser Leu Leu Glu Ile Ser Thr Val Glu Arg Gly Val Val Ser Leu Phe Gly Val Arg Ser Ala Leu Phe Val Ala Met Asn Ser Lys Gly Arg Leu Tyr Ala Thr Pro Ser Phe Gln Glu Glu Cys Lys Phe Arg Glu Thr Leu Leu Pro Asn Asn Tyr Asn Ala

Tyr Glu Ser Asp Leu Tyr Gln Gly Thr Tyr Ile Ala Leu Ser Lys Tyr
Gly Arg Val Lys Arg Gly Ser Lys Val Ser Pro Ile Met Thr Val Thr
His Phe Leu Pro Arg Ile,

or SEQ ID NO:3:

Met Val Val Pro Ser Pro Ala Gly Thr Arg Ala Asn Asn Thr Leu Leu
Asp Ser Arg Gly Trp Gly Thr Leu Leu Ser Arg Ser Arg Ala Gly Leu
Ala Gly Glu Ile Ala Gly Val Asn Trp Glu Ser Gly Tyr Leu Val Gly
Ile Lys Arg Gln Arg Arg Leu Tyr Cys Asn Val Gly Ile Gly Phe His
Leu Gln Val Leu Pro Asp Gly Arg Ile Ser Gly Thr His Glu Glu Asn
Pro Tyr Ser Leu Leu Glu Ile Ser Thr Val Glu Arg Gly Val Val Ser
Leu Phe Gly Val Arg Ser Ala Leu Phe Val Ala Met Asn Ser Lys Gly
Arg Leu Tyr Ala Thr Pro Ser Phe Gln Glu Glu Cys Lys Phe Arg Glu
Thr Leu Leu Pro Asn Asn Tyr Asn Ala Tyr Glu Ser Asp Leu Tyr Gln
Gly Thr Tyr Ile Ala Leu Ser Lys Tyr Gly Arg Val Lys Arg Gly Ser
Lys Val Ser Pro Ile Met Thr Val Thr His Phe Leu Pro Arg Ile.

**2.** A recombinant DNA sequence coding for an HST2 protein.

**3.** A recombinant DNA sequence according to claim 2, in which said HST2 protein is the protein claimed in claim 1.

**4.** A recombinant DNA sequence according to claim 2, having a sequence as shown in SEQ ID NO:4:

ATGGCCCTGG GACAGAAACT GTTCATCACT ATGTCCCGGG GAGCAGGACG TCTGCAGGGC
ACGCTGTGGG CTCTCGTCTT CCTAGGCATC CTAGTGGGCA TGGTGGTGCC CTCGCCTGCA
GGCACCCGTG CCAACAACAC GCTGCTGGAC TCGAGGGGCT GGGGCACCCT GCTGTCCAGG
TCTCGCGCGG GGCTAGCTGG AGAGATTGCC GGGGTGAACT GGGAAAGTGG CTATTTGGTG
GGGATCAAGC GGCAGCGGAG GCTCTACTGC AACGTGGGCA TCGGCTTTCA CCTCCAGGTG

CTCCCCGACG GCCGGATCAG CGGGACCCAC GAGGAGAACC CCTACAGCCT GCTGGAAATT
TCCACTGTGG AGCGAGGCGT GGTGAGTCTC TTTGGAGTGA GAAGTGCCCT CTTCGTTGCC
ATGAACAGTA AAGGAAGATT GTACGCAACG CCCAGCTTCC AAGAAGAATG CAAGTTCAGA
GAAACCCTCC TGCCCAACAA TTACAATGCC TACGAGTCAG ACTTGTACCA AGGGACCTAC
ATTGCCCTGA GCAAATACGG ACGGGTAAAG CGGGGCAGCA AGGTGTCCCC GATCATGACT
GTCACTCATT TCCTTCCCAG GATCTAA,

SEQ ID NO:5:

ATGTCCCGGG GAGCAGGACG TCTGCAGGGC ACGCTGTGGG CTCTCGTCTT CCTAGGCATC
CTAGTGGGCA TGGTGGTGCC CTCGCCTGCA GGCACCCGTG CCAACAACAC GCTGCTGGAC
TCGAGGGGCT GGGGCACCCT GCTGTCCAGG TCTCGCGCGG GGCTAGCTGG AGAGATTGCC
GGGGTGAACT GGGAAAGTGG CTATTTGGTG GGGATCAAGC GGCAGCGGAG GCTCTACTGC
AACGTGGGCA TCGGCTTTCA CCTCCAGGTG CTCCCCGACG GCCGGATCAG CGGGACCCAC
GAGGAGAACC CCTACAGCCT GCTGGAAATT TCCACTGTGG AGCGAGGCGT GGTGAGTCTC
TTTGGAGTGA GAAGTGCCCT CTTCGTTGCC ATGAACAGTA AAGGAAGATT GTACGCAACG
CCCAGCTTCC AAGAAGAATG CAAGTTCAGA GAAACCCTCC TGCCCAACAA TTACAATGCC
TACGAGTCAG ACTTGTACCA AGGGACCTAC ATTGCCCTGA GCAAATACGG ACGGGTAAAG
CGGGGCAGCA AGGTGTCCCC GATCATGACT GTCACTCATT TCCTTCCCAG GATCTAA,

or SEQ ID NO:6

ATGGTGGTGC CCTCGCCTGC AGGCACCCGT GCCAACAACA CGCTGCTGGA CTCGAGGGGC
TGGGGCACCC TGCTGTCCAG GTCTCGCGCG GGGCTAGCTG GAGAGATTGC CGGGGTGAAC
TGGGAAAGTG GCTATTTGGT GGGGATCAAG CGGCAGCGGA GGCTCTACTG CAACGTGGGC
ATCGGCTTTC ACCTCCAGGT GCTCCCCGAC GGCCGGATCA GCGGGACCCA CGAGGAGAAC
CCCTACAGCC TGCTGGAAAT TTCCACTGTG GAGCGAGGCG TGGTGAGTCT CTTTGGAGTG
AGAAGTGCCC TCTTCGTTGC CATGAACAGT AAAGGAAGAT TGTACGCAAC GCCCAGCTTC
CAAGAAGAAT GCAAGTTCAG AGAAACCCTC CTGCCCAACA ATTACAATGC CTACGAGTCA
GACTTGTACC AAGGGACCTA CATTGCCCTG AGCAAATACG GACGGGTAAA GCGGGGCAGC
AAGGTGTCCC CGATCATGAC TGTCACTCAT TTCCTTCCCA GGATCTAA.

5. A vector containing the recombinant DNA sequence as recited in claims 2, 3 or 4.

6. A host transformed with the vector of claim 5.

7. A method for preparing an HST2 protein comprising the steps of cultivating the transformed host of claim 6 under conditions suitable for the expression of the HST2 protein.

**8.** An HST2 protein made by the process of claim 7.

**Claims for the following Contracting States : ES, GR**

**1.** A method for preparing a recombinant DNA sequence coding for an HST2 protein having the following amino acid sequence, which comprises introducing the DNA sequence into cells:

SEQ ID NO:1:

Met Ala Leu Gly Gln Lys Leu Phe Ile Thr Met Ser Arg Gly Ala GLy
Arg Leu Gln Gly Thr Leu Trp Ala Leu Val Phe Leu Gly Ile Leu Val
Gly Met Val Val Pro Ser Pro Ala Gly Thr Arg Ala Asn Asn Thr Leu
Leu Asp Ser Arg Gly Trp Gly Thr Leu Leu Ser Arg Ser Arg Ala Gly
Leu Ala Gly Glu Ile Ala Gly Val Asn Trp Glu Ser Gly Tyr Leu Val
Gly Ile Lys Arg Gln Arg Arg Leu Tyr Cys Asn Val Gly Ile Gly Phe
His Leu Gln Val Leu Pro Asp Gly Arg Ile Ser Gly Thr His Glu Glu
Asn Pro Tyr Ser Leu Leu Glu Ile Ser Thr Val Glu Arg Gly Val Val
Ser Leu Phe Gly Val Arg Ser Ala Leu Phe Val Ala Met Asn Ser Lys
Gly Arg Leu Tyr Ala Thr Pro Ser Phe Gln Glu Glu Cys Lys Phe Arg
Glu Thr Leu Leu Pro Asn Asn Tyr Asn Ala Tyr Glu Ser Asp Leu Tyr
Gln Gly Thr Tyr Ile Ala Leu Ser Lys Tyr Gly Arg Val Lys Arg Gly
Ser Lys Val Ser Pro Ile Met Thr Val Thr His Phe Leu Pro Arg Ile,

SEQ ID NO:2:

Met Ser Arg Gly Ala Gly Arg Leu Gln Gly Thr Leu Trp Ala Leu Val
Phe Leu Gly Ile Leu Val Gly Met Val Val Pro Ser Pro Ala Gly Thr
Arg Ala Asn Asn Thr Leu Leu Asp Ser Arg Gly Trp Gly Thr Leu Leu
Ser Arg Ser Arg Ala Gly Leu Ala Gly Glu Ile Ala Gly Val Asn Trp
Glu Ser Gly Tyr Leu Val Gly Ile Lys Arg Gln Arg Arg Leu Tyr Cys
Asn Val Gly Ile Gly Phe His Leu Gln Val Leu Pro Asp Gly Arg Ile
Ser Gly Thr His Glu Glu Asn Pro Tyr Ser Leu Leu Glu Ile Ser Thr
Val Glu Arg Gly Val Val Ser Leu Phe Gly Val Arg Ser Ala Leu Phe

Val Ala Met Asn Ser Lys Gly Arg Leu Tyr Ala Thr Pro Ser Phe Gln
Glu Glu Cys Lys Phe Arg Glu Thr Leu Leu Pro Asn Asn Tyr Asn Ala
Tyr Glu Ser Asp Leu Tyr Gln Gly Thr Tyr Ile Ala Leu Ser Lys Tyr
Gly Arg Val Lys Arg Gly Ser Lys Val Ser Pro Ile Met Thr Val Thr
His Phe Leu Pro Arg Ile,

or SEQ ID NO:3:

Met Val Val Pro Ser Pro Ala Gly Thr Arg Ala Asn Asn Thr Leu Leu
Asp Ser Arg Gly Trp Gly Thr Leu Leu Ser Arg Ser Arg Ala Gly Leu
Ala Gly Glu Ile Ala Gly Val Asn Trp Glu Ser Gly Tyr Leu Val Gly
Ile Lys Arg Gln Arg Arg Leu Tyr Cys Asn Val Gly Ile Gly Phe His
Leu Gln Val Leu Pro Asp Gly Arg Ile Ser Gly Thr His Glu Glu Asn
Pro Tyr Ser Leu Leu Glu Ile Ser Thr Val Glu Arg Gly Val Val Ser
Leu Phe Gly Val Arg Ser Ala Leu Phe Val Ala Met Asn Ser Lys Gly
Arg Leu Tyr Ala Thr Pro Ser Phe Gln Glu Glu Cys Lys Phe Arg Glu
Thr Leu Leu Pro Asn Asn Tyr Asn Ala Tyr Glu Ser Asp Leu Tyr Gln
Gly Thr Tyr Ile Ala Leu Ser Lys Tyr Gly Arg Val Lys Arg Gly Ser
Lys Val Ser Pro Ile Met Thr Val Thr His Phe Leu Pro Arg Ile.

2. A method according to claim 1 wherein the recombinant DNA sequence has a nucleotide sequence of SEQ ID NO:4:

ATGGCCCTGG GACAGAAACT GTTCATCACT ATGTCCCGGG GAGCAGGACG TCTGCAGGGC
ACGCTGTGGG CTCTCGTCTT CCTAGGCATC CTAGTGGGCA TGGTGGTGCC CTCGCCTGCA
GGCACCCGTG CCAACAACAC GCTGCTGGAC TCGAGGGGCT GGGGCACCCT GCTGTCCAGG
TCTCGCGCGG GGCTAGCTGG AGAGATTGCC GGGGTGAACT GGGAAAGTGG CTATTTGGTG
GGGATCAAGC GGCAGCGGAG GCTCTACTGC AACGTGGGCA TCGGCTTTCA CCTCCAGGTG
CTCCCCGACG GCCGGATCAG CGGGACCCAC GAGGAGAACC CCTACAGCCT GCTGGAAATT
TCCACTGTGG AGCGAGGCGT GGTGAGTCTC TTTGGAGTGA GAAGTGCCCT CTTCGTTGCC
ATGAACAGTA AAGGAAGATT GTACGCAACG CCCAGCTTCC AAGAAGAATG CAAGTTCAGA

GAAACCCTCC TGCCCAACAA TTACAATGCC TACGAGTCAG ACTTGTACCA AGGGACCTAC
ATTGCCCTGA GCAAATACGG ACGGGTAAAG CGGGGCAGCA AGGTGTCCCC GATCATGACT
GTCACTCATT TCCTTCCCAG GATCTAA,

SEQ ID NO:5:

ATGTCCCGGG GAGCAGGACG TCTGCAGGGC ACGCTGTGGG CTCTCGTCTT CCTAGGCATC
CTAGTGGGCA TGGTGGTGCC CTCGCCTGCA GGCACCCGTG CCAACAACAC GCTGCTGGAC
TCGAGGGGCT GGGGCACCCT GCTGTCCAGG TCTCGCGCGG GGCTAGCTGG AGAGATTGCC
GGGGTGAACT GGGAAAGTGG CTATTTGGTG GGGATCAAGC GGCAGCGGAG GCTCTACTGC
AACGTGGGCA TCGGCTTTCA CCTCCAGGTG CTCCCCGACG GCCGGATCAG CGGGACCCAC
GAGGAGAACC CCTACAGCCT GCTGGAAATT TCCACTGTGG AGCGAGGCGT GGTGAGTCTC
TTTGGAGTGA GAAGTGCCCT CTTCGTTGCC ATGAACAGTA AAGGAAGATT GTACGCAACG
CCCAGCTTCC AAGAAGAATG CAAGTTCAGA GAAACCCTCC TGCCCAACAA TTACAATGCC
TACGAGTCAG ACTTGTACCA AGGGACCTAC ATTGCCCTGA GCAAATACGG ACGGGTAAAG
CGGGGCAGCA AGGTGTCCCC GATCATGACT GTCACTCATT TCCTTCCCAG GATCTAA,

or SEQ ID NO:6

ATGGTGGTGC CCTCGCCTGC AGGCACCCGT GCCAACAACA CGCTGCTGGA CTCGAGGGGC
TGGGGCACCC TGCTGTCCAG GTCTCGCGCG GGGCTAGCTG GAGAGATTGC CGGGGTGAAC
TGGGAAAGTG GCTATTTGGT GGGGATCAAG CGGCAGCGGA GGCTCTACTG CAACGTGGGC
ATCGGCTTTC ACCTCCAGGT GCTCCCCGAC GGCCGGATCA GCGGGACCCA CGAGGAGAAC
CCCTACAGCC TGCTGGAAAT TTCCACTGTG GAGCGAGGCG TGGTGAGTCT CTTTGGAGTG
AGAAGTGCCC TCTTCGTTGC CATGAACAGT AAAGGAAGAT TGTACGCAAC GCCCAGCTTC
CAAGAAGAAT GCAAGTTCAG AGAAACCCTC CTGCCCAACA ATTACAATGC CTACGAGTCA
GACTTGTACC AAGGGACCTA CATTGCCCTG AGCAAATACG GACGGGTAAA GCGGGGCAGC
AAGGTGTCCC CGATCATGAC TGTCACTCAT TTCCTTCCCA GGATCTAA.

3. A preparing method for a vector containing the recombinant DNA sequence as recited in claim 1 or 2 which comprises introducing the recombinant DNA sequence into a plasmid or phage.

4. A preparing method for a host transformed with the vector obtained in claim 3, which comprises introducing the vector into a host.

5. A method for preparing an HST2 protein which comprises the steps of cultivating the transformed host obtained in claim 4 under conditions suitable for the expression of the HST2 protein.

Fig. 1

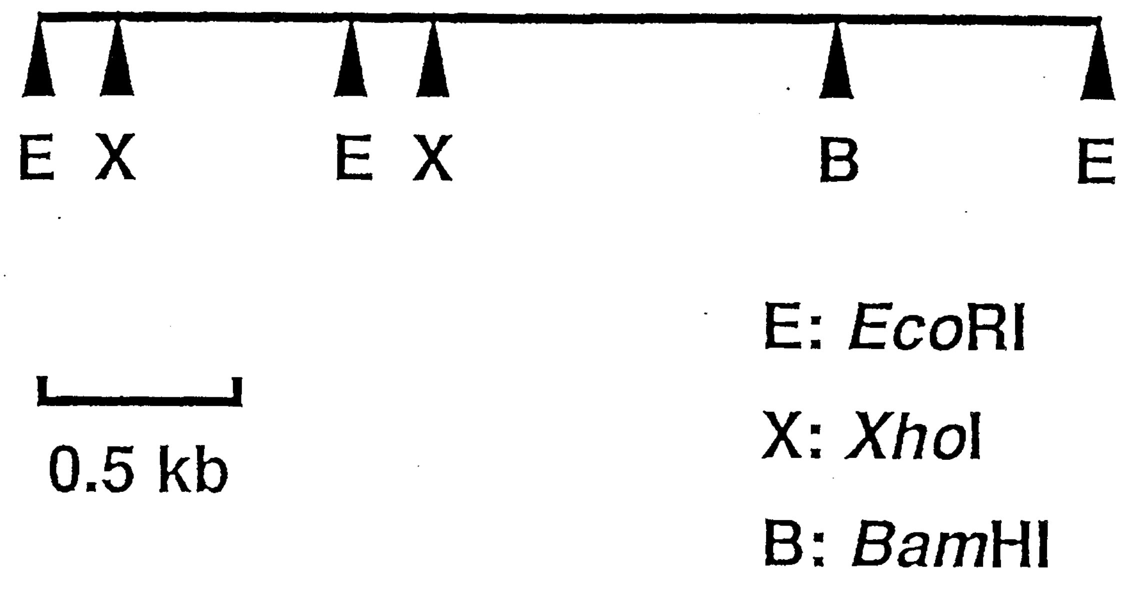

Fig. 2

1 A①TGGCCCTGG GACAGAAACT GTTCATCACT A②TGTCCCGGG GAGCAGGACG TATGCAGGGC 60
61 ACGCTGTGGG CTCTCGTCTT CCTAGGCATC CTAGTGGGCA③ TGGTGGTGCC CTCGCCTGCA 120
121 GGCACCCGTG CCAACAACAC GCTGCTGGAC TCGAGGGGCT GGGGCACCCT GCTGTCCAGG 180
181 TCTCGCGCGG GGCTAGCTGG AGAGATTGCC GGGGTGAACT GGGAAAGTGG CTATTTGGTG 240
241 GGGATCAAGC GGCAGCGGAG GCTCTACTGC AACGTGGGCA TCGGCTTTCA CCTCCAGGTG 300
301 CTCCCCGACG GCCGGATCAG CGGGACCCAC GAGGAGAACC CCTACAGCCT GCTGGAAATT 360
361 TCCACTGTGG AGCGAGGCGT GGTGAGTCTC TTTGGAGTGA GAAGTGCCCT CTTCGTTGCC 420
421 ATGAACAGTA AAGGAAGATT GTACGCAACG CCCAGCTTCC AAGAAGAATG CAAGTTCAGA 480
481 GAAACCCTCC TGCCCAACAA TTACAATGCC TACGAGTCAG ACTTGTACCA AGGGACCTAC 540
541 ATTGCCCTGA GCAAATACGG ACGGGTAAAG CGGGGCAGCA AGGTGTCCCC GATCATGACT 600
601 GTCACTCATT TCCTTCCCAG GATCTAA 627

Fig. 3

1 Met(①)AlaLeuGlyGlnLysLeuPheIleThrMet(②)SerArgGlyAlaGlyArgLeuGlnGly 20

21 ThrLeuTrpAlaLeuValPheLeuGlyIleLeuValGlyMet(③)ValValProSerProAla 40

41 GlyThrArgAlaAsnAsnThrLeuLeuAspSerArgGlyTrpGlyThrLeuLeuSerArg 60

61 SerArgAlaGlyLeuAlaGlyGluIleAlaGlyValAsnTrpGluSerGlyTyrLeuVal 80

81 GlyIleLysArgGlnArgArgLeuTyrCysAsnValGlyIleGlyPheHisLeuGlnVal 100

101 LeuProAspGlyArgIleSerGlyThrHisGluGluAsnProTyrSerLeuLeuGluIle 120

121 SerThrValGluArgGlyValValSerLeuPheGlyValArgSerAlaLeuPheValAla 140

141 MetAsnSerLysGlyArgLeuTyrAlaThrProSerPheGlnGluGluCysLysPheArg 160

161 GluThrLeuLeuProAsnAsnTyrAsnAlaTyrGluSerAspLeuTyrGlnGlyThrTyr 180

181 IleAlaLeuSerLysTyrGlyArgValLysArgGlySerLysValSerProIleMetThr 200

201 ValThrHisPheLeuProArgIle 208